# EUROPEAN PATENT APPLICATION

(11) **EP 1 806 160 A1**
(43) Date of publication of application: **11.07.2007**
(21) Application number: 05795617.9
(22) Date of filing: 19.10.2005
(51) Int. Cl.: A61M 25/00

(54) **CATHETER FOR MEDICAL TREATMENT**

(30) Priority: 22.10.2004 JP 2004307796
(71) Applicant: Kaneka Corporation, Kita-ku Osaka-shi, Osaka 530-8288 (JP)
(72) Inventor: FUKAYA, Kohei c/o Kaneka Corporation, Osaka 5660072 (JP)
(74) Representative: Gille Hrabal Struck Neidlein Prop Roos
(86) International application number: PCT/JP2005/019181
(87) International publication number: WO 2006/043582

(57) **Abstract**

The therapeutic catheter comprises a lumen communicating from the user's side end to the terminal tip part thereof, a main shaft having, at its tip, a guide wire lumen, a hub at its user's side end, and a slit in the guide wire lumen through its entire length, or the therapeutic catheter comprises amain shaft having a lumen communicating from the user's side end to the terminal tip part thereof, a guide wire shaft having , at its tip, a guide wire lumen, a hub at the user's side end of the main shaft, and a slit in the guide wire shaft through its entire length. The guide wire or the shaft of the other catheter playing the role of the guide wire can be rapidly disposed in the guide wire lumen through the slit.

## Description

### TECHNICAL FIELD

The present invention relates to therapeutic catheters such as catheters for percutaneous introduction into the body for injection of a medicine and suction catheters for withdrawal of debris (foreign matter), such as thrombus generated in blood vessel and atheroma liberated into blood vessel, out of the body under negative pressure applied from the catheter proximal end.

### BACKGROUND ART

When a body passage and vascular system such as blood vessel in the body is constricted or obstructed, traditionally percutaneous transluminal angioplasty (PTA, or PTCA: percutaneous transluminal coronary angioplasty) was practiced for expansion of the stricture or obstruction site of bloodvessel and improvement of blood flow in the peripheral blood vessel. These treatment methods have been practiced frequently in many medical institutions and used commonly in operation of such cases. In addition, there are many reports recently on stents that are used for preservation of the expanded stricture site.

The balloon catheter of PTA or PTCA is used in combination with a guide catheter and a guide wire, primarily for expansion of the stricture or obstruction site of blood vessel. In the angioplasty by using the balloon catheter, the distal end of a guide catheter is inserted from the femoral artery via aorta to the entrance of coronary artery; a guide wire penetrating the balloon catheter is sent over the stricture or obstruction site of blood vessel; the balloon catheter is then sent along the guide wire; the stricture or obstruction site is expanded, as the balloon is located and inflated at the stricture or obstruction site; and after treatment, the balloon is deflated and withdrawn out of the body. The balloon catheter is useful not only for treatment of the stricture or obstruction site of blood vessel, but also for many other medical applications, as it is inserted into the blood vessel or any other body cavity or lumen structure.

However, when obstruction of blood vessel is caused by thrombus formation, expansion of the obstructed site with a balloon catheter may result in liberation of the thrombus from the internal vascular wall and obstruction of the downstream peripheral blood vessel. In addition in expanding the stricture site in blood vessel, if the lesion contains a great amount of pultaceous plaque, expansion of the balloon catheter may lead to scattering of the pultaceous plaque (atheroma) in the lesion and obstruction of the downstream peripheral blood vessel. Even when the obstruction or stricture site is expanded, if the peripheral blood vessel is obstructed as described above, blood flow in the peripheral vessel declines, leading to a slow-flow or no-reflow state.

Commonly when the patient is in such a state, the surgeon waits until recovery of blood flow in coronary artery, which causes a problem of elongation of recovery period. Even if recovery of blood flow is accelerated by administration of a vasodilator such as nitroglycerol or local administration of a thrombolytic agent such as urokinase depending on the circumstance, there remains the problem that the period until recovery of blood flow is longer. When blood flow kinetics is undesirable because of severe peripheral obstruction, an additional means such as intra-aortic balloon pumping (IABP) may be used in combination. In addition to the thrombus-dissolving therapy, a method of removing thrombus by application of negative pressure from the catheter proximal end was also studied.

In the case of blood vessel obstruction or constriction of carotid or cerebral artery, peripheral obstruction caused by angioplasty with balloon catheter and stent results in termination of the blood flow into the brain and thus, leads to an ischemic state of the cerebral cells downstream of the obstruction site. Elongation of the cerebral ischemic state, which may result in death of cerebral cells and residual disorder, is very dangerous, and thus, sufficiently more caution should be given to prevent obstruction of peripheral blood vessel in angioplasty of cerebral or carotid artery than in angioplasty of other blood vessels.

For prevention of the obstruction of these peripheral blood vessels, practiced is angioplasty of the lesion in the state in which the peripheral blood vessel downstream of the lesion is previously occluded temporarily. A temporary occlusion balloon catheter is used as a means for occluding the blood vessel temporarily. In an embodiment of the temporary occlusion balloon catheter, the shaft of a temporary occlusion balloon catheter is used as the guide wire, and another therapeutic catheter, such as vasodilative balloon catheter, is inserted to the lesion along the shaft of the temporary occlusion balloon catheter. Then, the temporary occlusion balloon catheter is inflated in the peripheral area downstream of the lesion, and the lesion is treated while the blood flow is blocked. In addition, therapeutic catheters such as the catheter for injection of a medicine into the body and the suction catheter for withdrawal of debris (foreign matter), such as thrombus generated in blood vessel and atheroma liberated into blood vessel, out of the body under negative pressure applied from the catheter proximal end according to the invention are also used.

Then, the shaft of the temporary occlusion balloon catheter may be placed inside the guide wire lumen of another therapeutic catheter, and in such a case, the shaft should be sufficiently thinner than the internal diameter of the guide wire lumen of therapeutic catheter over the entire length of the shaft and thus, should have a diameter similar to that of common guide wires. The temporary occlusion balloon is inflated with an inflator connected to the catheter proximal end, but the inflator when connected prohibits operation of the other therapeutic catheter along the shaft of the temporary occlusion balloon catheter. Thus, the temporary occlusion balloon catheter should have a sealing mechanism for preservation of the expanded balloon even when the inflator (normally, used integrally with a connecting adapter, hereinafter referred to as adapter) at the proximal end is detached, and further, the external diameter of the sealing mechanism should be smaller than the guide wire lumen of the therapeutic catheter. The period of temporary occlusion is preferably as short as possible, but it takes much labor and a longer period to open and close the sealing mechanism and to feed and withdraw the fluid for inflation. It is because the adapter should be connected and disconnected for dilation with the temporary occlusion balloon catheter and use of a therapeutic catheter (in particular, multiple therapeutic catheters) in the temporarily occluded state. For example, removal of the atheroma generated by balloon inflation with a suction catheter while the peripheral blood vessel is occluded temporarily demands a great number of operations, including 1) connection of an adapter to a temporary occlusion balloon catheter, and inflation of the balloon and occlusion of blood flow, 2) removal of the adapter in that state, 3) insertion of a balloon catheter for inflation, 4) delivery and inflation thereof in the lesion, 5) removal of the balloon catheter for inflation, 6) insertion of a suction catheter, 7) suction operation, 8) removal of the suction catheter, 9) connection of an adaptor, and 10) deflation of the temporary obstruction balloon catheter.

Various adapters were studied for smoother operation of the sealing mechanism and for smoother inflation and deflation for improvement thereof, but the improvement is still insufficient. As for improvement of adapter, Patent Document 1 discloses a catheter occluding blood flow with a single expansion balloon and an adaptor for expansion and deflation of the expansion balloon. It only discloses that the adapter is connected detachably to the catheter proximal region, and apparently, the adaptor does not have a function to control the expanding unit. It seems that a surgeon operates the expanding unit directly in such a case, but it is fairly difficult to operate a thin shaft directly with fingers. In Patent Document 1, connection between the expanding unit and the catheter main body and fluid seal are performed by threading or friction. In the case of threading, it is very difficult to insert a thin expanding unit into a thin shaft with a screw in actual clinical settings, causing a problem of less effective operation. Alternatively in the case of friction, it is also difficult to operate the expanding unit, because the friction resistance made extremely higher for effective operation of the fluid seal. Operation of the hard-to-operate expanding unit leads to a problem that the thin expanding unit or the catheter shaft is broken. Also disadvantageous is that the operational period, in particular the period from treatment with therapeutic catheter to elimination of temporary occlusion, is very long. In addition in the case of adaptation by friction, there is a problem in production that the units should be produced accurately within very strict tolerance for effective operation of the fluid seal.

Patent Document 2 discloses a catheter having a low-profile valve and an inflation adapter for operation of the valve. It has a structure consisting of a housing having first and second regions and a catheter fixed in the housing, wherein the housing including the first and second regions is fixed by lock clipping. However, it was not possible to fix a catheter easily by one operation in such a structure, obviously causing a problem of clumsy operation in actual clinical settings.

Patent Document 3 discloses an anti-obstruction device containing an expandable unit used for prevention of occlusion and an adapter for operation of the expandable unit, which is connected to the proximal side of the anti-obstruction device and allows adjustment of the relative positions of the tension wire (operation unit in the invention) and the external shaft unit. However, the device has a structure in which two units are connected to each other like clam shell and bound to each other with a latch, and thus, has a problem that it is not possible to connect the catheter easily by a single operation, similarly to the adapter of Patent Document 2. Thus, there is still a problem that the operational period, in particular the period from treatment with therapeutic catheter to elimination of temporary occlusion, is very long.

Acceleration of catheter exchange by modification of the guide wire lumen was studied in Patent Document 4, but the guide wire lumen, which is designed there by considering the operation of removing the guide wire, has no slit extending over the entire length of the guide wire lumen, and accordingly, could not be removed unless the catheter is moved to the terminal of the guide wire. In addition, because there is no slit extending over the entire length of the guide wire lumen, it was not possible to place the guide wire in the guide wire lumen through the slit.
Alternatively, a method of inserting a guide wire into a catheter by twisting operation was studied in Patent Document 5, but there were problems such as inconvenience of operation because the rigidity of the distal region is significantly different from that of other regions, inconvenience of operation because the guide wire is not held tightly, and deterioration of the properties inherent to the catheter because of the presence of a guide wire in the catheter. Thus, such a catheter is not favorable as a suction catheter for cardiac and terminal blood vessels, which should be resistant to the vigorous bending force and give the maximum suction capacity, for example, because the catheter is less compatible with the guide wire inserted with many bending sites, and the suction efficiency declines significantly by deposition of the components to be removed such as thrombus on the guide wire present inside.

Mainly in the peripheral blood vessel, thrombus generated in blood vessel is removed by scraping with a coil- or balloon-shaped catheter for removal of thrombus, but a coil-shaped hub for control or balloon inflation is connected to the proximal end of the thrombus-removing catheter, and thus, it prohibits combined use of a suction catheter for removal under the negative pressure applied from the catheter proximal end, and forces use of a method of removing thrombus by dissecting the blood vessel.
Patent Document 1: Japanese Unexamined Patent Publication No. 2002-126093
Patent Document 2: Japanese Unexamined Patent Publication No. 2001-523535
Patent Document 3: Japanese Unexamined Patent Publication No. 2004-510524
Patent Document 4: Japanese Unexamined Patent Publication No. 63-288167
Patent Document 5: WO 01/068177

### Disclosure of the Invention

### Problems to be Solved by the Invention

As described above, it is needed to connect and disconnect an adapter for expansion and deflation of a balloon catheter in conventional therapeutic catheters, in particular when the balloon catheter is used as inserted in the guide wire lumen. Only adapter-removable balloon catheters were used in such applications. There was also a problem of elongated period needed for connecting/disconnecting the adapter, and many studies for shortening the periodwas performed only fruitlessly. In addition, conventional thrombus-removing catheters, which have a fixed hub, could not be used in combination with another therapeutic catheter.

### Means to Solve the Problems

An object of the present invention is to provide a therapeutic catheter produced from selected raw materials by a new production method in a new therapeutic catheter structure that allows rapid placement of a guide wire or the shaft of another catheter practically having a role of guide wire in the guide wire lumen, independently of connection of an adapter. An aspect of the present invention is a therapeutic catheter, characterized by including a main shaft having a lumen extending from the proximal end region to the distal end region and a guide wire lumen in the distal region for controlling a guide wire, and a hub connected to the proximal end thereof, wherein the guide wire lumen has a slit extending over the entire length of the guide wire lumen. Another aspect of the present invention is a therapeutic catheter, characterized by including a main shaft having a lumen extending from the proximal end region to the distal end region inside, a guide wire shaft having a guide wire lumen for controlling guide wire inside in the distal end region of the main shaft, and a hub connected to the proximal end of the main shaft, wherein the guide wire shaft has a slit extending over the entire length of the guide wire lumen. These aspects of the present invention, which allows rapid placement of a guide wire or the shaft of another catheter practically having a role of guide wire from the slit into the guide wire lumen, solve the problems described above.

The material for the guide wire lumen preferably has a structure containing an overlapping region in the slit region. It more preferably has a structure containing a guide assisting insertion of the guide wire in the area close to the slit region or a stopper for prevention of opening of the guide wire lumen in the slit region. It also preferably has a radiopaque marker indicating the position of the distal end of catheter in X-ray photography in the area close to the boundary between the guide wire lumen and the main lumen or close to the area where the guide wire shaft and the main shaft are connected to each other. The structure having an overlapping region and the guide/stopper structure may be formed alone or in combination.

The therapeutic catheter according to the present invention is most effective, when it is used as a suction catheter removing fluid out of the body by the negative pressure applied from the catheter proximal end.

### Advantageous Effects of the Invention

The guide wire or the shaft of another catheter practically having a role of guide wire should be inserted from the opening at the distal or proximal end of the guide wire lumen for conventional therapeutic catheters. In the present invention, it is possible to shorten the treatment period by making the guide wire and the shaft of another catheter practically having a role of guide wire more easily, rapidly placed in the guide wire lumen. In particular in the method of treating blood vessel by using the shaft of a temporary occlusion balloon catheter as guide wire, it is possible to shorten the period needed for occluding blood vessel drastically, when compared with a treatment method of removing the atheroma generated by the balloon inflation described above with a suction catheter, because it is possible to eliminate operation for connecting/disconnecting the adapter for expansion or deflation of the temporary occlusion balloon by inserting the temporary-occlusion balloon catheter into an expansion balloon catheter previously, for example by 1) connecting an adapter to a temporary occlusion balloon catheter, expanding the balloon and occluding blood flow, 2) sending the balloon catheter for inflation to the lesion in the state, and expanding the catheter, 3) removing the balloon catheter for inflation, 4) placing a temporary occlusion balloon catheter in the guide wire lumen of the therapeutic catheter according to the present invention, i.e., suction catheter, and sending it to the lesion, 5) performing suction, 6) removing the suction catheter, and 7) deflating the temporary obstruction balloon catheter, and thus it is possible to place the temporary-occlusion balloon catheter in the guide wire lumen of the therapeutic catheter according to the present invention. The present invention eliminates the operation of connecting and disconnecting an adapter associated with expansion and deflation of the temporary occlusion balloon catheter and thus, allows simplification of the adapter system.

### BRIEF DESCRIPTION OF THE DRAWINGS

Figure 1 is a side view illustrating the distal end of an embodiment of the therapeutic catheter according to the invention.
Figure 2 is a sectional view as seen along the line A-A in Figure 1.
Figure 3 is a sectional view illustrating another embodiment of the therapeutic catheter according to the invention having the overlapping structure of the material for the guide wire lumen in the slit region, similar to that shown in Figure 2.
Figure 4 is a sectional view illustrating another embodiment of the therapeutic catheter according to the invention having an overlapping structure of the material for the guide wire lumen in the slit region and a guide assisting insertion of the guide wire into the slit region, similar to that shown in Figure 2.
Figure 5 is a sectional view illustrating another embodiment of the therapeutic catheter according to the invention having an overlapping structure of the material for the guide wire lumen in the slit region, a guide assisting insertion of the guide wire into the slit region, and a stopper preventing opening of the guide wire lumen, similar to that shown in Figure 2.

### EXPLANATION OF REFERENCES

101 Main shaft
102 Distal region of the main shaft for guide wire lumen or guide wire shaft
103 Slit
104 Radiopaque marker
201 Main shaft
202 Guide wire shaft
203 Slit
204 Radiopaque marker
205 Guide wire lumen
206 Main shaft lumen
301 Main shaft
302 Guide wire shaft
303 Slit
304 Radiopaque marker
305 Guide wire lumen
306 Main shaft lumen
401 Main shaft
402 Guide wire shaft
403 Slit
404 Radiopaque marker
405 Guide wire lumen
406 Main shaft lumen
407 Guide assisting insertion of guide wire
501 Main shaft
502 Guide wire shaft
503 Slit
504 Radiopaque marker
505 Guide wire lumen
506 Main shaft lumen
507 Guide assisting insertion of guide wire
508 Stopper preventing opening of guide wire lumen

### BEST MODE OF CARRYING OUT THE INVENTION

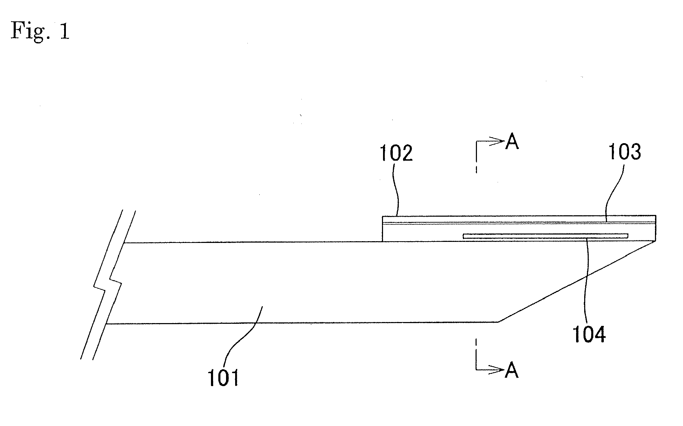

Hereinafter, the best embodiments of the therapeutic catheter according to the invention will be described. The present invention relates to a therapeutic catheter, characterized by including a main shaft having a lumen extending from the proximal end region to the distal end region and a guide wire lumen for controlling guide wire in the distal end region, and a hub at the proximal end region thereof, wherein the guide wire lumen has a slit extending over the entire length of the guide wire lumen. Figure 1 is a side view of the distal end of a therapeutic catheter according to the invention having a main shaft 101 having a lumen extending from the proximal end region to the distal end region, a guide wire lumen in the distal end region for controlling guide wire, and a hub at the proximal end region. As shown in Figure 1 , there is a slit 103 extending over the entire length of the guide wire lumen formed in the distal end region 102 of the main shaft for the guide wire lumen. The guide wire lumen may be formed integrally with the main shaft, but a guide wire shaft having a guide wire lumen for controlling guide wire may be formed as it is connected to the distal end of a main shaft having a lumen extending from the proximal end region to the distal end region inside. In such a case, the guide wire shaft having a guide wire lumen inside is represented by 102 in Figure 1. If the shape of slit region is modified as will be described below, the guide wire shaft is preferably formed and processed separately from the main shaft, and connected to the main shaft after the slit region is processed. The connection method is not particularly limited, but a welding method is generally favorable because the prof ile becomes smaller. Laser welding is most favorable because it allows connection of the guide wire shaft without adverse effects such as deformation on the slit area. Numeral 104 in Figure 1 represents a radiopaque marker.

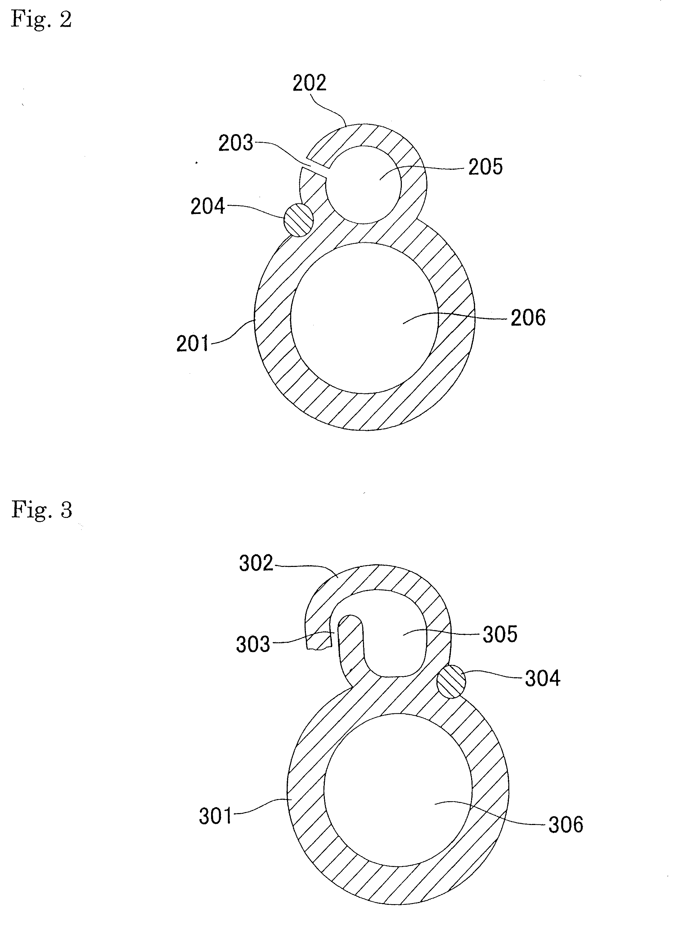

Figure 2 is a sectional view as seen along the line A-A in Figure 1. In the Figure, numeral 201 represents a main shaft; 202, a guide wire shaft; 203, a slit; 204, a radiopaque marker; 205, aguidewirelumen; and206, amain shaft lumen. As described above, the guide wire shaft 202 may be a guide wire lumen integrally welded on the distal region of the main shaft 201. The material for the main shaft or the guide wire shaft is not particularly limited, and favorable examples thereof for use include various thermoplastic elastomers such polyethylene, polyamide, polyurethane,polyamide elastomer,and polyester elastomer. As for the properties of the guide wire shaft, insufficient hardness results in insufficient guide wire-holding power, and thus, use of a material having a Shore hardness of 55D or more is preferable, and use of a material having a Shore hardness of 63D or more is particularly preferable.

The length of the guide wire lumen in the catheter axial direction is to be decided, by taking into consideration integrally the balance between the inconvenience in handling the guide wire through the slit when the lumen is longer and the lower guide wire-holding power when it is shorter, the flexibility of the entire catheter, and the convenience of operation, but it is preferably a length in the range of 10 to 50 times, more preferably in the range of 15 to 30 times, larger than the nominal diameter of the guide wire.

The angle and the position of the slit are also not particularly limited, and should be decided, by considering the convenience of operating the guide wire through the slit, guide wire-holding power, and other properties of the catheter integrally, but it is mostly, preferably welded in the direction almost parallel to the catheter axis direction, and the guide wire may be placed more easily when the slit is formed in a direction slightly inclined from the axis as needed. The position of the slit in the circumference direction is also not particularly limited, but it is preferable to form the slit on the guide wire shaft at a position inclined from the line connecting the main shaft and the guide wire shaft as shown in Figures 1 and 2, from the points of convenience in controlling the guide wire and the guide wire-holding power.

The shape and structure of the slit region are also not particularly limited, and various shapes and structures are possible for overall improvement in the convenience of controlling the guide wire through the slit, guide wire-holding power, and other properties of the catheter, but, as shown in Figure 3, a structure of guide wire lumen material having an overlapping area in the slit region is preferable for improvement of the guide wire-holding power. The width of the overlapping region is not particularly limited, but is preferably in the range of 5% to 30% with respect to the circumferential length of the guide wire lumen internal surface, for better balance between the convenience of operation and the holding force. In the Figure, numeral 301 represents a main shaft; 302, a guide wire shaft; 303, a slit; 304, a radiopaque marker; 305, a guide wire lumen; and 306, a main shaft lumen.

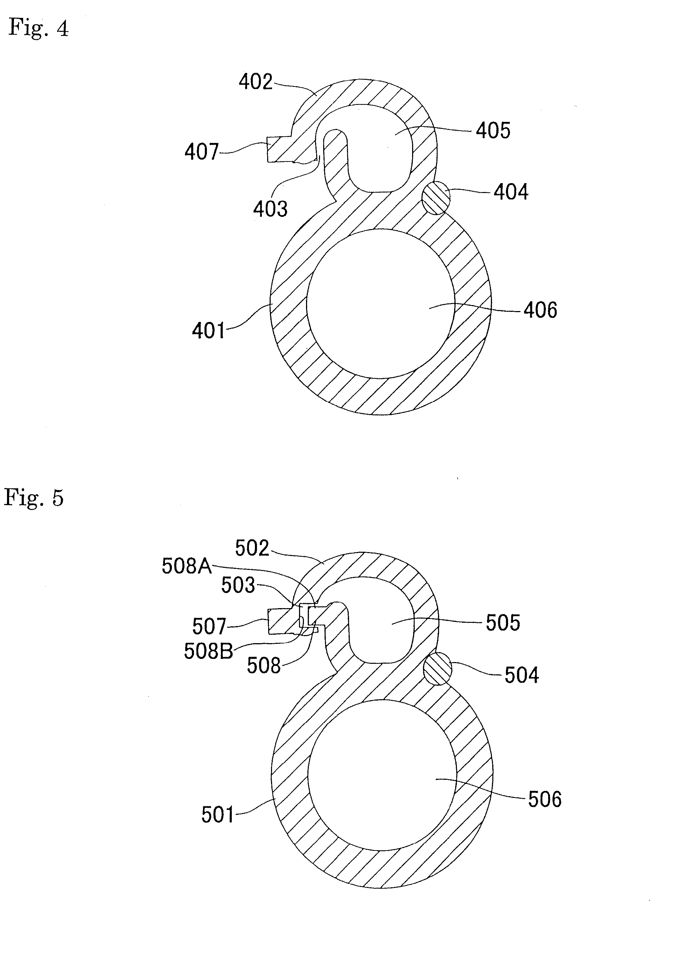

As shown in Figure 4, it is also possible to improve the convenience in controlling the guide wire through the slit, by forming a protruded guide 407 assisting insertion of the guide wire through the slit in the region outside the slit-overlapping region. The shape of the guide is preferably a shape that assists deformation toward opening of the slit and insertion of the guide wire when the guide wire is pressed onto the slit. The other configuration is the same as that shown in Figure 3 and thus, detailed description thereof is omitted. In the Figure, numeral 401 represents a main shaft; 402, a guide wire shaft; 403, a slit; 404, a radiopaque marker; 405, a guide wire lumen; and 406, a main shaft lumen.

As shown in Figure 5, it is possible to increase the guide wire-holding power, by forming a guide 507 similar to that described above and additionally a stopper structure 508 for preventing opening of the guide wire lumen in the slit region. The structure of the stopper is also not limited, but, as shown in Figure 5, the structure having a raised wall (stopper raised wall 508A) and a groove (stopper groove 508B) allowing connection to the wall on the corresponding position of the external overlapping region inside the overlapping region of the material for the slit is preferable, from the points of the balance among easiness of production, convenience of operation, and guide wire-holding power. The other configuration is the same as those shown in Figures 3 and 4, and thus, detailed description thereof is omitted. In the Figure, numeral 501 represents a main shaft; 502, a guide wire shaft; 503, a slit; 504, a radiopaque marker; 505, a guide wire lumen; 506, a main shaft lumen; and 507, a guide wire.

Preferably, at least one radiopaque marker indicating the position of the distal end of catheter in X-ray photography is connected to the catheter distal region, and, as shown in Figures 1 to 5, it is preferably connected to the area close to the boundary between the guide wire lumen and the main lumen or the area where the guide wire shaft and the main shaft are connected to each other, for softening the catheter distal end. As shown in Figures 3 to 5, it is preferably connected to the wall of the guide wire lumen opposite to the slit for favorable guide wire-holding power.

Hereinafter, typical examples of the present invention will be described in detail with reference to Examples and Comparative Examples. However, the present invention is not limited by the following Examples.

### Example 1

A main shaft consisting of a proximal shaft, a middle shaft and a distal shaft was prepared. A polyimide tube having an external diameter of 1.5 mm, an internal diameter of 1.3 mm, and a length of 1, 100 mm was prepared by dipping in a polyamide acid varnish and used as the proximal shaft. A tube of a low-density polyethylene (LF480 M, Japan Polychem Corporation) having an external diameter of 1.5 mm, an internal diameter of 1. 2 mm, and a length of 300 mm was prepared by extrusion molding and used as the middle shaft. Similarly, a tube of a low-density polyethylene (LF480 M, Japan Polychem Corporation) having an major diameter of approximately 1.9 mm, a minor diameter approximately 1.5 mm, and a length 10.5 mm and also having two lumens therein was prepared by extrusion molding and used as the distal shaft. A rod-shaped radiopaque marker having an elliptical cross section previously coated with low-density polyethylene (LF480 M, Japan Polychem Corporation) was placed at a position corresponding to the boundary of the two lumens of the distal shaft and fixed thereon by heating while mandrels respectively having external diameters of 0.4 mm and 1.15 mm are inserted respectively in the lumens. The internal diameters of the lumens of the distal shaft are respectively 0.4 mm and 1.15 mm. A slit was formed in the lumen having an internal diameter of 0.4 mm of the distal shaft, in the direction almost parallel with the axial direction with an ultrasonic wave cutter. The proximal shaft was narrowed at one end by heat drawing, and the narrowed region was inserted into the middle shaft and bonded with a two-liquid-mixing urethane bonding agent. Further, the lumen having an internal diameter of 1.15 mm of distal shaft and the middle shaft were heat-welded to each other, giving a main shaft. Mandrels for protection were inserted into both lumens during welding for preservation of the guide wire lumen and the lumen extending from the proximal end region to the distal end region.

A polycarbonate (Makloron 2658, manufactured by Bayer) hub prepared by injection molding was bonded to the proximal end of the main shaft with a two-liquid-mixing urethane bonding agent. A therapeutic catheter having a cross section represented by Figure 2 in the distal end region, i.e., a suction catheter for use with a guide wire having a nominal diameter of 0.014 inch, was obtained by bonding a rod-shaped radiopaque marker having an elliptical cross section to the area close to the boundary between the guide wire lumen and the main lumen with a two-liquid-mixing urethane bonding agent.

### Example 2

A main shaft consisting of a proximal shaft and a distal shaft was prepared. A polyimide tube having an external diameter of 1.5 mm, an internal diameter of 1.3 mm, and a length of 110 cm was prepared by dipping in a polyamide acid varnish and used as the proximal shaft. A tube of a polyamide elastomer (PEBAX 6333, ATOCHEM) having an external diameter of 1.5 mm, an internal diameter of 1.2 mm, and a length of 30 cm was prepared by extrusion molding and used as the distal shaft. The proximal shaft was narrowed at one end by heat drawing, and the narrowed region was inserted into the proximal region and bonded with a two-liquid-mixing urethane bonding agent, to give a main shaft. The most distal region of the main shaft was cut diagonally with a razor.

A tube having an external diameter of 0.68 mm, an internal diameter of 0 . 47 mm, and a length of 5. 5 mm was prepared by using a polyamide elastomer having a Shore hardness of 72D (PEBAX 7233, ATOCHEM) by extrusion molding; a slit was formed in the tube with an ultrasonic wave cutter in the direction almost parallel with the axial direction; and the tube was welded in the direction parallel with the distal region of the main shaft while a mandrel having an internal diameter of 0.40 mm was inserted therein and the tube was pressed from outside. Then, a rod-shaped radiopaque marker having an elliptical cross section previously coated with PEBAX 6333 was placed on the wall opposite to the slit in the area close to the boundary between the guide wire lumen and the main lumen and fixed there simultaneously with welding of the guide wire lumen with the main lumen. After bonding, there was a region of the material for the guide wire lumen overlapping each other in the slit region, and the width thereof was approximately 0.2 mm (approximately 16% with respect to the circumferential length of the guide wire lumen internal surface). A polycarbonate (Makloron 2658, manufactured by Bayer) hub prepared by injection molding was connected to the proximal end of the main shaft with a two-liquid-mixing urethane bonding agent, to give a therapeutic catheter having the cross section represented by Figure 3 in the distal region, i.e., a suction catheter for use with a guide wire having a nominal diameter of 0.014 inch.

### Example 3

A main shaft consisting of a proximal shaft and a distal shaft was prepared. A polyimide tube having an external diameter of 1.5 mm, an internal diameter of 1.3 mm, and a length of 110 cm was prepared by dipping in a polyamide acid varnish and used as the proximal shaft. A polyamide elastomer (PEBAX 6333, ATOCHEM) tube having an external diameter of 1. 5 mm, an internal diameter of 1.2 mm, and a length of 30 cm was prepared by extrusion molding and used as the distal shaft. The proximal shaft was narrowed at one end by heat drawing, and the narrowed region was inserted into the distal shaft and bonded with a two-liquid-mixing urethane bonding agent, to give a main shaft. The most distal region of the main shaft was cut diagonally with a razor.

A tube having an external diameter of 0. 70 mm, an internal diameter of 0.47 mm, and a length of approximately 300 mm was prepared by extrusion molding of a polyamide elastomer having a Shore hardness of 63D (PEBAX 6333 , ATOCHEM), and a raw material tube for guide wire lumen was prepared by allowing the tube to penetrate under heat into a dice having an external diameter of 0.65 mm that has a dent on the internal surface for forming a guide assisting insertion of the guide wire while a mandrel having an internal diameter of 0.43 mm was inserted in the tube. The tube was cut into pieces of 10.5 mm in length, and a slit was formed in the raised side wall in the direction almost parallel with the axial direction with an ultrasonic wave cutter; and the tube was welded in the direction parallel with the distal region of the main shaft, while a mandrel having an internal diameter of 0.40 mm was inserted therein and the tube was pressed from outside. Then, a rod-shaped radiopaque marker having an elliptical cross section previously coated with PEBAX 6333 was placed on the wall opposite to the slit in the area close to the boundary between the guide wire lumen and the main lumen and fixed there, simultaneously with welding of the guide wire lumen with the main lumen. After bonding, there was a region of the material for the guide wire lumen overlapping each other in the slit region, and the width thereof was approximately 0.2 mm (approximately 16% with respect to the circumferential length of the guide wire lumen internal surface). A polycarbonate (Makloron 2658, manufactured by Bayer) hub prepared by injection molding was connected to the proximal end of the main shaft with a two-liquid-mixing urethane bonding agent , to give a therapeutic catheter having the cross section represented by Figure 4 in the distal region, i.e., a suction catheter for use with a guide wire having a nominal diameter of 0.014 inch.

### Example 4

A main shaft consisting of a proximal shaft and a distal shaft was prepared. A polyimide tube having an external diameter of 1.5 mm, an internal diameter of 1.3 mm, and a length of 110 cm was prepared by dipping in a polyamide acid varnish and used as the proximal shaft. A polyamide elastomer (PEBAX 6333, ATOCHEM) tube having an external diameter of 1. 5 mm, an internal diameter of 1. 2 mm, and a length of 30 cm was prepared by extrusion molding and used as the distal shaft. The proximal shaft was narrowed at one end by heat drawing, and the narrowed region was inserted into the distal shaft and bonded with a two-liquid-mixing urethane bonding agent, to give a main shaft. The most distal region of the main shaft was cut diagonally with a razor.

A tube of a polyamide elastomer having a Shore hardness of 55D (PEBAX 5533, manufactured by ATOCHEM) having an external diameter of 0.75 mm, an internal diameter of 0.53 mm, and a length of approximately 300 mm was prepared by extrusion molding, and a raw material tube for guide wire lumen was prepared by allowing the tube to penetrate under heat into a dice having an external diameter of 0.65 mm that has a groove for forming a guide for inserting the guide wire and a raised wall for forming its neighboring stopper raised wall (508A) on the internal surface, while a mandrel having an internal diameter of 0.48 mm was inserted in the tube. The tube was cut into pieces of 10.5 mm in length, and a stopper groove (508B) was formed by heating the raised wall while a mandrel having an internal diameter of 0.45 mm and containing a raised wall for forming a stopper groove is placed inside. A slit was formed between the raised walls of the guide and the stopper raised wall in the direction almost parallel with the axial direction with an ultrasonic wave cutter; the mandrel was replaced with another mandrel having an internal diameter of 0.40 mm; and the guide and the stopper wall are connected to each other in the direction parallel with the distal region of the main shaft while it is pressed from outside. Then, a rod-shaped radiopaque marker having an elliptical cross section previously coated with PEBAX 6333 was placed on the wall opposite to the slit in the area close to the boundary between the guide wire lumen and the main lumen and fixed there, simultaneously with welding of the guide wire lumen with the main lumen. After bonding, there was a region of the material for the guide wire lumen overlapping each other in the slit region, and the width thereof was approximately 0.2 mm (approximately 16% with respect to the circumferential length of the guide wire lumen internal surface). A polycarbonate (Makloron 2658, manufactured by Bayer) hub prepared by injection molding was connected to the proximal end of the main shaft with a two-liquid-mixing urethane bonding agent, to give a therapeutic catheter having the cross section represented by Figure 5 in the distal region, i.e., a suction catheter for use with a guide wire having a nominal diameter of 0.014 inch.

### Example 5

A tube having an external diameter of 1.7 mm, an internal diameter of 1.4 mm, and a length of 45 cm was prepared with a polyamide elastomer (PEBAX 7233, manufactured by ATOCHEM) by extrusion molding and used as the main shaft. The most distal region of the main shaft was cut diagonally with a razor. A tube of a polyamide elastomer having a Shore hardness of 63D (PEBAX 6333 , manufactured by ATOCHEM) having an external diameter of 0.79 mm, an internal diameter of 0.61 mm, and a length of 11 mm was prepared by extrusion molding; a slit was in the direction almost parallel with the axial direction with an ultrasonic wave cutter; the mandrel was replaced with another mandrel having an internal diameter of 0.50 mm; and the tube was connected to the main shaft in the direction parallel with the distal region of the main shaft while it is pressed from outside. Then, a rod-shaped radiopaque marker having an elliptical cross section previously coated with PEBAX 6333 was placed on the wall opposite to the slit in the area close to the boundary between the guide wire lumen and the main lumen and fixed there, simultaneously with welding of the guide wire lumen with the main lumen. After bonding, there was a region of the material for the guide wire lumen overlapping each other in the slit region, and the width thereof was approximately 0.2 mm (approximately 13% with respect to the circumferential length of the guide wire lumen internal surface). A polycarbonate (Makloron 2658, manufactured by Bayer) hub prepared by injection molding was connected to the proximal end of the main shaft with a two-liquid-mixing urethane bonding agent, to give a therapeutic catheter having the cross section represented by Figure 3 in the distal region, i.e., a suction catheter for use with a guide wire having a nominal diameter of 0.018 inch.

### (Comparative Example 1)

In Comparative Example 1, a commercially available suction catheter (Thrombuster 7F, manufactured by Kaneka Medix Corp.) consisting of a main shaft of a proximal polyimide tube having an external diameter of 1.5 mm, an internal diameter of 1.3 mm, and a length of 1, 100 mm and a distal polyethylene tube having a external diameter of 1.5 mm, an internal diameter of 1.2 mm, and a length of 300 mm, and a polyethylene guide wire tube connected to the distal region of the main shaft having an external diameter of 0.56 mm, an internal diameter of 0.43 mm, and a length 10 mm was used.

### (Evaluation of the convenience of operation in pig coronary artery)

A guiding catheter (Launcher 7FHS, manufactured by Medtronic Japan) was guided through a sheath inserted from the pig femoral artery into the coronary artery; a commercially available occlusion catheter (guard wire, manufactured by Medtronic Japan) was sent to LCX#13 by the guiding catheter; a PTCA balloon catheter (Aerocrossfighter 3.0 mm, manufactured by Kaneka Medix Corp.) for inflation was placed in the proximal region of the occlusion catheter; and an adapter was connected to the proximal sealing mechanism of the occlusion catheter. The occlusion catheter was inflated to a diameter of 4 mm in that state, allowing expansion of the PTCA catheter in the area upstream of the occlusion catheter (at 6 atm for 30 seconds) and then removed; a suction catheter was inserted, allowing suction (30 cc syringe), and then removed; the occlusion catheter was allowed to shrink; and the period until blood flow was determined. The convenience in operating each of the suction catheters obtained in Examples 1 to 4 and the suction catheter obtained in Comparative Example 1 was determined at N=3 (test number).

The blood-reflow period of the catheter obtained in Example 1 was 174±12 seconds, that of Example 2, 182±21 seconds; that of Example 3, 140±17 seconds; and that of Example 4, 155±25 seconds. The blood-reflow period of the catheter in Comparative Example was 207±25 seconds, indicating superiority of the catheters obtained in Examples.

### (Percutaneous removal of thrombus in pig femoral artery)

A thrombus-removing catheter including a Ni-Ti alloy tube having an external diameter of 0.018 inch (0.45 mm) as the main shaft, a polyurethane soft balloon formed in the distal region, and a hub connected to the proximal region was prepared. The catheter was inserted into the femoral artery through a sheath inserted from the pig femoral artery, for collection of thrombus . A thrombus-removing catheter was placed through the slit into the guide wire lumen of the suction catheter obtained in Example 5 and inserted into the femoral artery for withdrawal of the collected thrombus. There was no conventional suction catheter that allowed use of a balloon-typed thrombus-removing catheter like a guide wire.

## Claims

1. A therapeutic catheter, **characterized by** including a main shaft having a lumen extending from the proximal end region to the distal end region and a guide wire lumen for controlling a guide wire in the distal region, and a hub connected to the proximal end thereof, wherein the guide wire lumen has a slit extending over the entire length of the guide wire lumen.

2. A therapeutic catheter, **characterized by** including a main shaft having a lumen extending from the proximal end region to the distal end region inside, a guide wire shaft having a guide wire lumen for controlling guide wire inside in the distal end region of the main shaft, and a hub connected to the proximal end of the main shaft, wherein the guide wire shaft has a slit extending over the entire length of the guide wire lumen.

3. The therapeutic catheter according to Claim 1 or 2, wherein the material for the guide wire lumen has a structure in which there is an overlapping region in the slit region.

4. The therapeutic catheter according to any one of Claims 1 to 3, wherein a guide for assisting insertion of the guide wire is formed in the area close to the slit region.

5. The therapeutic catheter according to any one of Claims 1 to 4, wherein the slit region has a stopper structure of preventing opening of the guide wire lumen.

6. The therapeutic catheter according to Claim 1 or 2, wherein a radiopaque marker indicating the position of the catheter distal region in X-ray photography is placed in the area close to the boundary between the guide wire lumen and the main lumen or to the area where the guide wire shaft and the main shaft are connected to each other.

7. The therapeutic catheter according to any one of Claims 1 to 6, wherein the therapeutic catheter is a suction catheter of withdrawing and removing by application of negative pressure from the catheter proximal end.
